# EUROPÄISCHE PATENTSCHRIFT

(11) **EP 4 185 219 B1**
(45) Veröffentlichungstag und Bekanntmachung des Hinweises auf die Patenterteilung: **06.03.2024**
(21) Anmeldenummer: 21754742.1
(22) Anmeldetag: 23.07.2021
(51) Int. Cl.: A61B 17/16

(54) **MEDIZINISCHES INSTRUMENT MIT KUGEL- ODER GLEITLAGER ALS GENERATOR**
MEDICAL INSTRUMENT HAVING A BALL BEARING OR SLIDING BEARING AS A GENERATOR
INSTRUMENT MÉDICAL DOTÉ D'UN ROULEMENT À BILLES OU D'UN PALIER LISSE COMME GÉNÉRATEUR

(30) Priorität: 23.07.2020 DE 102020119427
(43) Veröffentlichungstag der Anmeldung: 31.05.2023
(73) Patentinhaber: Aesculap AG, 78532 Tuttlingen (DE)
(72) Erfinder: BÜRK, André, 78056 Villingen-Schwenningen (DE); LENZENHUBER, Frederick, 78532 Tuttlingen (DE)
(74) Vertreter: Winter, Brandl - Partnerschaft mbB
(86) Internationale Anmeldenummer: PCT/EP2021/070714
(87) Internationale Veröffentlichungsnummer: WO 2022/018273

(56) Entgegenhaltungen:
- EP-A1- 2 704 295
- DE-U1-202007 010 974

## Beschreibung

Die Offenbarung betrifft ein vorzugsweise motorisch oder manuell betriebenes medizinisches Instrument zum vorzugsweise rotatorischen Betreiben eines wahlweise darin aufgenommenen, chirurgischen Werkzeugs mit wenigstens einem Lager oder einer Lagereinheit, vorzugsweise der Kugel- oder Gleitlager-Bauform mit Stromgenerator-Funktion.

### Stand der Technik

Medizinische Instrumente, insbesondere chirurgische Motorensysteme, d.h. chirurgische Instrumente, welche handbetrieben werden oder einen Motor aufweisen, wie etwa Fräs-, Bohr- oder Schraubhandstücke, werden zunehmend mit neuen Funktionen ausgestattet, die eine Übermittlung, Weiterleitung bzw. Übertragung von elektrischen Signalen erfordern. Derartige neue Funktionen können beispielsweise sein, dass
- eine Temperatur an einer Spitze eines Werkzeugs (Bohr- oder Fräswerkzeug) über Temperatursensoren ermittelt wird,
- Kräfte/Drehmomente beim Fräsen oder Bohren über Dehnungsmessstreifen ermittelt werden, oder
- ein in das medizinische Instrument (Bohr-/Fräshandstück) eingesteckter Werkzeugtyp über Sensoren oder Antennen, beispielsweise RFID respektive NFC-Ausleseantennen, identifiziert wird.
- Ebenfalls können Vibrationssensoren oder Neigungssensoren und Sensoren vielfältiger Art mit unterschiedlichen Funktionen vorgesehen sein.

Diese neuen Funktionen haben gemeinsam, dass sie für eine Übermittlung, Weiterleitung bzw. Übertragung von Daten eine elektrische Verbindung zu einem Steuergerät benötigen.

Eine solche elektrische Verbindung wird bisher beispielsweise durch Anbringen bzw. Einfügen von Signalleitungen in das Fräshandstück selbst realisiert. Dabei werden elektrische Signale über einzelne, isolierte Signallitzen übertragen, welche in einem separaten Kanal durch den Schaft des medizinischen Instruments (Bohr-/Fräshandstücks) verlaufen und sich bis hin zu einer Spitze/einem distalen Ende des medizinischen Instruments (Bohr-/Fräshandstücks) erstrecken. Aufgrund der kompakten Bauart und des begrenzten Bauraums von chirurgischen/medizinischen Instrumenten wie Bohr- oder Fräshandstücken bedeutet ein Einbringen bzw. Einfügen von herkömmlichen Signalleitungen in das Instrument jedoch, dass ein Außendurchmesser des Instruments, insbesondere eines, das Instrument in Richtung distal (weg vom Benutzer/Anwender) wahlweise verlängernden Instrumentenschaftes vergrößert werden muss, da ein zusätzlicher Kanal für die isolierten Signallitzen erforderlich wird.

Diese mangelnde Integration der Signallitzen in den bestehenden Aufbau der medizinischen Instrumente ist aus Anwendersicht jedoch nicht wünschenswert und als negativ zu beurteilen, da sich für den Anwender/ Chirurg dadurch ein Sichtzugang zu einer Operationsstelle im Patienten verschlechtert und das medizinische Instrument (Handstück) seine Eignung insbesondere für enge OP-Zugänge verliert. Weiterhin zeichnet sich der bestehende Aufbau durch eine erschwerte Montage, schwierige Anschlussmöglichkeiten und eine komplizierte Anbindung von mehreren Signalgebern bei einer Verwendung als Bus aus.

Der Stand der Technik hat außerdem immer den Nachteil, dass eine Energieversorgung der oben erwähnten Baugruppen bzw. Sensorik in Form von Leitungen durch das medizinische Instrument (Handstück ggf. mit Instrumentenschaft) zu den Baugruppen bereitgestellt werden muss. Hierfür ist immer eine elektrische Verbindung zu einem übergeordneten Stromgeber im Gesamtsystem bzw. einem entsprechenden Steuergerät vorgesehen.

Es kann daher ein Bedarf bestehen zum Bereitstellen von Konzepten für zumindest teilweise autonome Stromversorgung durch das medizinische Instrument, insbesondere des Handstücks, selbst.

Ein konkretes Beispiel für den bisherigen Stand der Technik stellt die DE 10 2007 012 586 B3 mit einer dentalen Bearbeitungsmaschine dar.

Weiterer Stand der Technik ist darüber hinaus aus der DE 20 2007 010 974 U1 und der EP 2 704 295 A1 bekannt.

### Kurzbeschreibung der Erfindung

Es ist demzufolge die Aufgabe der vorliegenden Offenbarung, die Nachteile aus dem Stand der Technik zu vermeiden oder wenigstens zu mildern. Insbesondere soll elektrische Energie bei Verwendung des medizinischen Instruments geerntet/rückgewonnen werden (englisch: Energy Harvesting).

Diese Aufgabe wird durch ein (motorisch/von Hand betriebenes) medizinisches Instrument mit den Merkmalen des Anspruchs 1 gelöst. Vorteilhafte Ausgestaltungen des offenbarten medizinischen Instruments sind Gegenstand der Unteransprüche.

Der Grundgedanke der vorliegenden Offenbarung besteht demzufolge im Wesentlichen darin, zumindest ein/eine Lager/Lagereinheit/Lagereinrichtung vorzugsweise der Kugel- oder Gleitlagerbauart mit einer Stromgeneratorfunktion auszubilden, derart, dass bei einem manuellen oder motorischen Betrieb des medizinischen Instruments an dem/der Lager/Lagereinheit/Lagereinrichtung mechanische Energie in elektrische Energie umgewandelt/rückgewonnen wird, die zur wenigstens teilweisen Versorgung insbesondere Instrumenten-eigener Sensorik verwendbar ist.

Es hat sich herausgestellt, dass insbesondere bei medizinischen Instrumenten der Handinstrumentenbauart vorzugsweise für minimalinvasive Eingriffe ein Instrumenteneffektor (Zange, Schere, Pinzette, Schneidwerkzeug, etc.) über einen vergleichsweise langgestreckten Instrumentenschaft mit einem Hand- oder Griffstück gekoppelt ist, wobei am oder nahe dem Effektor eine Sensorik vorgesehen ist, über die bestimmte Messgrößen abgegriffen werden müssen. Eine derartige Sensorik muss über Leiterkabel mit elektrischer Energie versorgt werden, welche ausgehend vom Griffstück über/durch den Instrumentenschaft bis zum Effektor geführt sein müssen.

Wird hingegen, wie in der vorliegenden Erfindung vorgesehen, das/die zumindest eine Lager/Lagereinheit/Lagereinrichtung insbesondere innerhalb des Instrumentenschafts als Stromgenerator benutzt, kann die Kabelführungsstrecke gegenüber dem Stand der Technik deutlich verringert werden.

Konstruktiv lässt sich der vorstehende Grundgedanke u.a. dadurch umsetzen, indem beispielsweise im Fall eines Kugellagers der die Kugeln haltende/lagernde (Kugel-) Käfig mit wenigstens einem oder mehreren umfangsbeabstandeten Permanentmagneten ausgerüstet/ausgebildet ist und eine zumindest den (Kugel-) Käfig (wenigstens abschnittsweise) umgebende (bezüglich des Käfigs ortsfeste) Hülle (Lagerring oder der Instrumentenschaft selbst) als/mit zumindest eine/einer Spule ausgebildet/versehen ist. Die Hülle kann dabei als eine dem/der Lager/Lagereinheit/Lagereinrichtung zugeordnete (in diese integrierte) zumindest den Käfig radial außenseitig umgebende separate Hülse (Lagerring) oder ein das/die Lager/Lagereinheit/Lagereinrichtung (radial außenseitig) abstützender Gehäuseabschnitt des medizinischen Instruments oder ein das medizinische Instrument axial in Richtung distal (weg vom Anwender) verlängernder Instrumenten-(Verlängerungs-)schaft sein, der bevorzugt wahlweise (in unterschiedlichen Längen verfügbar) an das Instrument (Handstück/Handgriff) ankoppelbar ist oder einstückig mit diesem ausgebildet ist.

Im Fall eines Gleitlagers ist es beispielsweise möglich, eine radial äußere Lagerbuchse (entspricht der vorstehend genannten Hülle) axialabschnittsweise mit zumindest einer Spule auszubilden und eine axial-drehbar in der Lagerbuchse gleitgelagerte Aufnahmehülse beispielsweise zur Aufnahme eines Werkzeugschafts oder einer Antriebsstange (entspricht dem vorstehend genannten Käfig) mit zumindest einem oder mehreren umfangsbeabstandeten Permanentmagneten zu versehen.

Im konkreten Fall eines/einer Kugellagers/Kugellagereinheit ist es vorteilhaft, zwei axial beabstandete Kugellager mit jeweils einem Innen- und einem Außenring über einen gemeinsamen Kugelkäfig zu koppeln und eine Distanzhülse zumindest axial zwischen den beiden Außenringen der axial beabstandeten Kugellager vorzusehen, welche unter Ausbildung der vorstehend genannte Hülle zumindest den Kugelkäfig axial zwischen den beiden Außenringen umgibt und somit die Außenringe axial beabstandet. Eine derart aufgebaute Lagereinheit ist bevorzugt in den, ein medizinisches Instrument (Handstück) wahlweise axial (distal) verlängernden Instrumentenschaft zur Aufnahme beispielsweise einer Drehmomentübertragungsstange (Getriebestange) oder eines Werkzeugschafts eingesetzt/einsetzbar.

In abstrakteren Worten ausgedrückt, wird ein in eine für eine medizinisches Instrument (Instrument/Handstück) vorgesehene Lagerhülse (Instrumenten-Verlängerungsschaft/Instrumentengehäuse) eingesetztes oder einsetzbares Lagerungs-Bauteil (Kugelkäfig) bereitgestellt. Das Bauteil (Kugelkäfig) hat zumindest einen zylindrischen Abschnitt. Der zylindrische Abschnitt weist eine Magnetisierung auf. Diese kann in Form einer vollständigen Magnetisierung oder einer Magnetanordnung in Form eines einzelnen oder einzelner mehrere Permanentmagnete, nämliche Permanentmagnete sein. Die Magnetisierung ist vorgesehen bzw. ausgebildet, beim Betrieb des medizinischen Instruments, in oder entgegen der Drehrichtung eines Motors des medizinischen Instruments mitgedreht zu werden. Ferner ist die Magnetisierung vorgesehen bzw. ausgebildet dabei, nämlich basierend auf dem Mitdrehen, in einer in der Lagerhülse (Instrumenten-Verlängerungsschaft/Instrumentengehäuse) vorgesehenen Spule einen Strom zu induzieren.

Durch die Magnetisierung in dem Bauteil (Kugelkäfig), das bei Verwendung in das medizinische Instrument (Instrument/Handstück) eingesetzt ist, und in Reaktion auf die durch die Motordrehung erfolgte Bauteildrehung verändert sich das Magnetfeld, welches durch geeignete Aufbringung einer Spule in der Lagerhülse (Instrumenten-Verlängerungsschaft/Instrumentengehäuse) entsprechend effektiv darin einen Strom induziert. Hierdurch lässt sich folglich eine zumindest teilweise autonome Stromversorgung von an die Spule angeschlossenen Geräten, Sensoren etc. erzielen.

Das Bauteil (Kugelkäfig) kann hierin abstrakt verstanden werden. Zum einen kann es in der Lagerhülse (Instrumenten-Verlängerungsschaft/Instrumentengehäuse) bereits eingesetzt sein, zum anderen für den Gebrauch eingesetzt werden. Im eingesetzten Zustand kann das Bauteil (Kugelkäfig) so in der Lagerhülse (Instrumenten-Verlängerungsschaft/Instrumentengehäuse) angebracht sein, dass es zumindest als Reaktion auf die Motordrehung vorzugsweise in dieselbe Richtung mitdreht. Dies kann eine direkte oder indirekte Übersetzung sein. Dies kann abhängig von Lage und Anbringung des Bauteils (Kugelkäfigs) in der Lagerhülse (Instrumenten-Verlängerungsschaft/Instrumentengehäuse) sein.

Der zylindrische Abschnitt kann entweder ein Teil des Bauteils (Kugelkäfigs) oder das gesamte Bauteil (Kugelkäfig) sein. Der zylindrische Abschnitt kann ein zylindrischer Hohlkörper oder ein zylindrischer Vollkörper sein. In einem Beispiel können über 90% des Bauteils (Kugellagers) der zylindrische Abschnitt sein. In einem anderen Beispiel können über 50% des Bauteils (Kugelkäfigs) der zylindrische Abschnitt sein.

Das Bauteil (Kugelkäfig) kann ein oder mehrere Permanentmagneten aufweisen. Die Magnetisierung kann durch die einen oder mehreren Permanentmagnete ausgebildet sein. Die einen oder mehreren Permanentmagnete können an dem zylindrischen Abschnitt angebracht sein. Somit stellen die einen oder mehreren Permanentmagneten die Magnetisierung an dem Bauteil (Kugelkäfig) bereit.

Jeweilige Nord- und Südpole der einen oder mehreren Permanentmagnete können in Radialrichtung des zylindrischen Abschnitts nebeneinander angeordnet sein. Zum Beispiel liegen entweder entsprechende Nordpole oder Südpole weiter von einem Zentrum des zylindrischen Abschnitts entfernt bzw. in Radialrichtung nach außen. Die einen oder mehreren Permanentmagnete können jeweils eine Ausdehnung in Längsrichtung des zylindrischen Abschnitts aufweisen. Die Ausdehnung in Längsrichtung des zylindrischen Abschnitts kann zumindest ein Viertel, vorzugsweise etwa eine Hälfte, einer Ausdehnung des zylindrischen Abschnitts in Längsrichtung des zylindrischen Abschnitts sein.

Ein Außenmantel bzw. eine Außenseite des zylindrischen Abschnitts des Bauteils (Kugelkäfigs) kann eine oder mehrere Aussparungen bzw. Vertiefungen aufweisen. In die Aussparung kann sich zumindest ein Teil der jeweiligen einen oder mehreren Permanentmagneten befinden. Der andere Teil der jeweiligen einen oder mehreren Permanentmagneten kann überstehen. Die einen oder mehreren Permanentmagneten können mittels Kleber/Klebstoff, in den einen oder mehreren Aussparungen bzw. Vertiefungen fixiert sein. Der Klebstoff kann beispielsweise auf Basis von Silikon, insbesondere ein Silikonverguss, hergestellt sein. Der Silikonverguss kann auch dazu dienen, die einen oder mehreren Permanentmagnete vor Korrosion zu schützen. Als Lösungsmittel kann eine Mischung aus Ester und aliphatischen Lösungmitteln in dem Klebstoff enthalten sein. Ebenso können die einen oder mehreren Permanentmagneten in die einen oder mehreren Aussparungen bzw. Vertiefungen entsprechend eingepasst sein. Dafür können die einen oder mehreren Aussparungen bzw. Vertiefungen als Passung ausgebildet sein. Somit kann eine einfache Integration der Magnetisierung bereitgestellt werden.

In einer bevorzugten Ausführungsform können mehrere Permanentmagnete auf gegenüberliegenden Seiten des zylindrischen Abschnitts angeordnet sein. Dies kann einem Winkelabstand entlang eines Umfangs des zylindrischen Abschnitts von 180° entsprechen. Ferner kann, bei drei Permanentmagneten, ein Winkelabstand der mehreren Permanentmagnete zueinander von etwa 120° vorgesehen sein. Bei vier Permanentmagneten kann ein Winkelabstand der mehreren Permanentmagnete zueinander von etwa 90° vorgesehen sein (usw.). Diese Anordnungen der Permanentmagnete kann eine effektive Induktion von Strom in der in der Lagerhülse vorgesehenen Spule bewirken.

In einer vorteilhaften Ausführungsform kann das Bauteil (Kugelkäfig) ein zylindrisches Rohr oder axialabschnittsweise ein Vollkörper sein. Das zylindrische Rohr oder der Vollkörper hat vorzugsweise an seinen jeweiligen Endbereichen Aufnahme-Hohlräume für die Kugeln der beiden axial beabstandeten Kugellager. Dabei sei an dieser Stelle darauf hingewiesen, dass anstelle von Kugeln auch Nadeln oder Zylinderrollen vorgesehen sein können.

Weiter vorzugsweise können die beiden axial beabstandeten Kugellager, jeweils bestehend aus dem Innen- und Außenring, den radial dazwischen gelagerten Kugeln, der gemeinsame, einzige Kugelkäfig sowie die radial äußere, den Kugelkäfig zwischen den Außenringen umgreifende Hülle/Distanzhülse zu einer einzigen patronenartigen Einheit zusammengefasst sein, welche einfach beispielsweise in den Instrumenten-Verlängerungsschaft, das Instrumentengehäuse oder auf einen Werkzeugschaft ein- /aufgesetzt wird.

Die oben definierte Aufgabe wird bei dem gattungsgemäßen Instrument in noch anderen Worten dadurch gelöst, dass ein Instrumenten-Verlängerungsschaft für ein medizinisches Instrument (Instrument/Handstück) bzw. zur wahlweisen Montage an dem medizinischen Instrument (Handstück) bereitgestellt wird. Der Instrumenten-Verlängerungsschaft kann an das medizinische Instrument angebracht werden oder Teil derselben sein. Der Instrumenten-Verlängerungsschaft weist eine an einer Innenumfangsseite flächig angeordnete mäanderförmige oder spiralförmige Spule auf, die entweder unmittelbar im/am Schaft oder in/an der separaten Distanzhülse ausgebildet ist, die in den Schaft eingesetzt ist. Die Spule ist, wie vorstehend bereits ausgeführt wurde, ausgebildet, beim Betrieb des medizinischen Instruments, basierend auf einer Magnetisierung eines bevorzugt in Drehrichtung eines Motors des medizinischen Instruments mitgedrehten Bauteils (Kugelkäfigs) einen Strom zu erzeugen.

Nach einer oder mehreren Ausführungsformen können Signalleitungen bzw. Signalbahnen besser in den bestehenden Aufbau von medizinischen Instrumenten, wie chirurgische (Motor-) Instrumente (Bohr-/Fräshandstücke), integriert werden, ohne dabei die Abmessungen des medizinischen Instruments bzw. des in dem medizinischen Instrument vorgesehenen Bauteils (Kugelkäfigs) zu verändern, also ohne beispielsweise den Außendurchmesser des medizinischen Instruments bzw. des den Kugelkäfig umgebenden Instrumenten-Verlängerungsschafts zu vergrößern. Zum Beispiel wird eine neue Bauweise eines Kugel-/Wälzlagers und des Instrumenten-Verlängerungsschafts bereitgestellt, welche eine Weiterleitung/ Übertragung/ Übermittlung von elektrischen Signalen durch das Kugel-/Wälzlager und den Instrumenten-Verlängerungsschaft hindurch, sowie zwischen diesen Bauelementen ermöglicht, wobei die Abmessungen der Bauelemente (Kugel-/Wälzlager und Instrumenten-Verlängerungsschaft) unverändert bleiben, so dass der Außendurchmesser des medizinischen Instruments nicht vergrößert wird. Das medizinische Instrument behält demnach seine kompakte Bauart und der bestehende Bauraum wird geeignet ausgenutzt. Das Wälzlager, insbesondere Kugellager, kann zur vorzugsweise multidirektionalen Weiterleitung bzw. Übertragung von elektrischen Signalen eingerichtet sein und dazu zumindest eine in das Kugel-/Wälzlager integrierte Signalleitung bzw. Signalbahn aufweisen.

Das Wälzlager ist, wie vorstehend bereits angedeutet wurde, nicht auf ein Kugellager beschränkt, d.h. es sollen auch beliebige andere Wälzlager wie etwa Zylinderrollenlager, Nadellager, Kegelrollenlager, Tonnenlager, Toroidalrollenlager, etc. hierin umfasst sein. Das Kugellager stellt vorliegend jedoch die bevorzugte Ausführungsform eines Wälzlagers dar. Weiter bevorzugt ist, dass das Kugellager ein Mikro-Kugellager ist. Das Wälz-/Kugellager ist bevorzugt zur Verwendung in dem medizinischen Instrument, insbesondere dem chirurgischen Instrument/Handstück, insbesondere dem Bohr-/Fräshandstück, und noch besonderer dem Instrumenten-Verlängerungsschaft eingerichtet bzw. geeignet bzw. vorgesehen.

Beispielsweise können die Signalleitung bzw. Signalbahn in dem Außenring des Wälzlagers/ Kugellagers integriert sein.

Bevorzugt ist das Wälzlager/ Kugellager (insbesondere der Außenring des Wälzlagers/ Kugellagers) aus einem nichtleitenden Material hergestellt. Weiter bevorzugt handelt es sich bei dem Material des Wälzlagers/ Kugellagers (des Außenrings) um ein hartes Material. Als besonders geeignet hat sich dabei Keramik herausgestellt.

Die Signalleitung bzw. Signalbahn ist bevorzugt aus einem (gut) leitenden Material, insbesondere Kupfer, Silber oder Gold, hergestellt.

Eine vorteilhafte Ausführungsform sieht zumindest eine Signalleitung, insbesondere Signallitze vor, welche in eine in dem Wälzlager/ Kugellager, insbesondere dem Außenring des Wälzlagers/ Kugellagers, vorgesehene Bohrung, die sich über eine gesamte axiale Länge des Wälzlagers/ Kugellagers erstreckt, eingesetzt ist. Bevorzugt ist die Signalleitung bzw. Signallitze in der Bohrung axial fixiert.

Dementsprechend weist das Wälzlager/ Kugellager bzw. der Außenring des Wälzlagers/ Kugellagers bevorzugt zumindest eine feine Bohrung auf. Beispielsweise kann der Durchmesser der Bohrung kleiner als 0,2 mm sein. Bevorzugt ist der Durchmesser etwa im Bereich von 0,1 mm. Als Herstellungs-/ bzw. Fertigungsverfahren für eine derart feine Bohrung hat sich insbesondere Mikrolaserbohren als geeignet herausgestellt.

Entsprechend ist auch der Durchmesser der Signalleitung bzw. Signallitze bevorzugt kleiner als 0,2 mm, weiter bevorzugt etwa im Bereich von 0,1 mm.

Ein axiales Fixieren der Signalleitung bzw. Signallitze in der Bohrung kann beispielsweise über ein plastisches Verformen von axialen Enden der Signalleitung bzw. Signallitze realisiert werden. Hier hat sich insbesondere ein Verstemmen als geeignet herausgestellt. Alternativ kann die Bohrung auch zunächst metallisiert werden (bevor die Signalleitung bzw. Signallitze eingesetzt wird) und die axiale Fixierung kann über eine Klebeverbindung oder eine Hartlotverbindung erfolgen.

Beispielsweise kann die Signalleitung in axialer Richtung des Wälzlagers/ Kugellagers (über den Außenring) hervorstehen/ herausstehen, insbesondere an beiden Seiten/ axialen Enden des Wälzlagers/ Kugellagers, so dass die Signalleitung zur Kontaktierung bzw. Steckverbindung mit einem anderen Bauelement des medizinischen Instruments, insbesondere mit der Distanzhülse als separates Bauteil oder als Bestandteil des Instrumenten-Verlängerungsschafts, eingerichtet ist. Bevorzugt steht die Signalleitung etwa 0,1 bis 0,3 mm über den Außenring hervor bzw. heraus, um diese mit den auf der Keramik eingebrachten Bahnen verlöten zu können.

Bevorzugt ist eine Vielzahl von Signalleitungen bzw. Signalbahnen, beispielsweise zwei, drei, vier, fünf, sechs oder mehr, vorgesehen. Die Signalleitungen bzw. Signalbahnen können grundsätzlich beliebig über den Umfang des Wälzlagers/ Kugellagers/ des Außenrings verteilt sein. Es ist auch denkbar, den gesamten Kreisring des Wälzlagers/ Kugellagers (des Außenrings) auszunutzen. Demnach können die Signalleitungen auch gleichmäßig über den Kreisring verteilt sein.

Eine Obergrenze für die Anzahl von Signalleitungen bzw. Signalbahnen ergibt sich bevorzugt aus der Größe des Wälzlagers/ Kugellagers. Insbesondere hat sich herausgestellt, dass (insbesondere bei dem bevorzugten Bohrungsdurchmesser bzw. Signalleitungsdurchmesser) für das Verhältnis von Außendurchmesser D (in mm) des Kugellagers zur Anzahl der Bohrungen bzw. Signalleitungen N gelten soll: D/N > 0,1. Das Vorsehen einer Vielzahl von Signalleitungen bzw. Signalbahnen um den Umfang verteilt kann dazu führen, dass der Übergangswiderstand reduziert wird (Stichwort: parallele Mehrleitertechnik).

In einer oder mehreren Ausführungsformen kann die Distanzhülse und insbesondere der Instrumenten-Verlängerungsschaft zur, vorzugsweise multidirektionalen, Weiterleitung bzw. Übertragung von elektrischen Signalen eingerichtet sein und dazu zumindest eine in die Distanzhülse und insbesondere in den Instrumenten-Verlängerungsschaft integrierte Signalleitung bzw. Signalbahn aufweisen.

Weiter bevorzugt ist die Distanzhülse und insbesondere der Instrumenten-Verlängerungsschaft zur Verwendung in dem medizinischen Instrument, insbesondere einem chirurgischen Handstück, insbesondere einem Bohr-/Fräshandstück, eingerichtet bzw. geeignet bzw. vorgesehen.

Bevorzugt ist die Distanzhülse ggf. auch der Instrumenten-Verlängerungsschaft aus einem nichtleitenden Material hergestellt. Weiter bevorzugt handelt es sich bei dem Material der Distanzhülse ggf. auch des Instrumenten-Verlängerungsschafts um ein hartes Material. Als besonders geeignet hat sich dabei Keramik herausgestellt. Die Signalleitung bzw. Signalbahn ist bevorzugt aus einem (gut) leitenden Material, insbesondere Kupfer, Silber oder Gold, hergestellt.

Die Distanzhülse und insbesondere der Instrumenten-Verlängerungsschaft ist bevorzugt zur Weiterleitung bzw. Übertragung von elektrischen Signalen in einer Axialrichtung zwischen einem ersten axialen Ende und einem zweiten axialen Ende des Instrumenten-Verlängerungsschafts und/oder in einer Radialrichtung zwischen einer Innenmantelfläche und einer Außenmantelfläche des Instrumenten-Verlängerungsschafts eingerichtet.

Zum Beispiel weist eine Außenmantelfläche der Distanzhülse oder eine Innenmantelfläche des Instrumenten-Verlängerungsschafts zumindest eine Rille/ einen Kanal auf, welche/r sich über eine gesamte axiale Länge der Distanzhülse oder des Instrumenten-Verlängerungsschafts erstreckt. Bevorzugt ist in der Rille/ dem Kanal die Signalbahn bzw. Signalleitung vorgesehen bzw. angeordnet. In anderen Worten befindet sich in der Rille/ dem Kanal leitendes Material. Dadurch kann erreicht werden, dass elektrische Signale an der Außenmantelfläche/ im Außenbereich der Distanzhülse und/oder an der Innenmantelfläche des Instrumenten-Verlängerungsschafts abgegriffen, sowie weitergeleitet bzw. übertragen werden können.

Der zumindest eine Kanal bzw. die zumindest eine Rille ist bevorzugt fein bzw. filigran ausgebildet und durch Schleifen oder Gravieren, insbesondere Lasergravieren, hergestellt. Der Kanal bzw. die Rille wird bevorzugt metallisiert und mit dem gut leitenden Material beschichtet, um die Signalleitung bzw. Signalbahn auszubilden.

In einem Beispiel ist die Signalbahn bzw. Signalleitung bezüglich der Außenmantelfläche der Distanzhülse und/oder bezüglich der Innenmantelfläche des Instrumenten-Verlängerungsschafts nach innen versetzt, so dass die Signalbahn bzw. Signalleitung nur in einem unteren/inneren Bereich der Rille vorgesehen ist. In anderen Worten ist die Signalbahn bzw. Signalleitung bevorzugt vollständig in der Rille/ dem Kanal versenkt, so dass die (äußere/innere) Mantelfläche der Distanzhülse oder des Instrumenten-Verlängerungsschafts von der Signalbahn bzw. Signalleitung in der Radialrichtung der Distanzhülse/des Instrumenten-Verlängerungsschafts beabstandet ist. Somit schließt die Signalbahn/ Signalleitung vorzugsweise nicht bündig mit der Außen- oder Innenmantelfläche ab, sondern befindet sich weiter innen. Insbesondere wenn mehr als eine Signalbahn bzw. Signalleitung vorgesehen ist, wird damit erreicht, dass die einzelnen Signalbahnen bzw. Signalleitungen elektrisch voneinander getrennt sind. Dies ist insbesondere erforderlich, da der Instrumenten-Verlängerungsschaft des Instruments (Bohr-/Fräshandstücks), in welchem die Distanzhülse bevorzugt eingesetzt werden soll und an welchem die Distanzhülse direkt anliegt, auch aus Metall sein kann.

Es ist zweckmäßig, wenn über der Signalbahn bzw. Signalleitung ein Isolator angeordnet ist. In anderen Worten kann die angesprochene elektrische Trennung der Signalbahnen bzw. Signalleitungen voneinander verbessert werden, wenn zusätzlich ein Isolator vorgesehen ist. Der Isolator kann beispielsweise ein Einlegeteil sein, insbesondere aus Silikon. Alternativ kann der Isolator beispielsweise auch mittels einer Klebeschicht realisiert werden. Durch die zusätzliche Isolation wird das medizinische Instrument, insbesondere das Bohr-/Fräshandstück, in/an welches die Distanzhülse insbesondere der Instrumenten-Verlängerungsschaft an/einzusetzen ist, unempfindlicher gegenüber eindringende leitende Flüssigkeiten (z.B. eine Kochsalzlösung).

In einem oder mehreren Beispielen kann eine Innenmantelfläche der Distanzhülse zumindest eine Signalbahn bzw. Signalleitung aufweisen. Wenn zusätzlich oder alternativ an der Innenfläche der Distanzhülse Signalleitungen bzw. Signalbahnen vorgesehen sind, können in dem Innenbereich elektrische Signale abgegriffen, sowie weitergeleitet bzw. übertragen werden. Beispielsweise können an der Innenmantelfläche metallisierte Bahnen (zumindest eine metallisierte Bahn) vorgesehen sein.

In einem weiteren Beispiel kann eine an einer Innenmantelfläche der Distanzhülse vorgesehene Signalbahn bzw. Signalleitung elektrisch leitend mit einer an einer Außenmantelfläche der Distanzhülse vorgesehenen Signalbahn bzw. Signalleitung verbunden sein. Beispielsweise kann die Distanzhülse feine Bohrungen (Mikrobohrungen) aufweisen, welche sich in der Radialrichtung der Distanzhülse erstrecken, und über welche eine Signalleitung bzw. Signalbahn an der Innenmantelfläche elektrisch leitend mit einer Signalleitung bzw. Signalbahn an der Außenmantelfläche verbindbar/ verbunden ist (beispielsweise mittels leitendem Material in der Bohrung). In anderen Worten verläuft die Bohrung (Mikrobohrung) bevorzugt zwischen der Rille/ dem Kanal an der Außenmantelfläche und der Signalleitung bzw. Signalbahn an der Innenmantelfläche.

Mit anderen Worten werden wie in der Leiterplattentechnik Durchkontaktierungen geschaffen, welche gleichzeitig ebenso als Lötaugen fungieren können. Somit könnten auch bedrahtete Komponenten in das System integriert werden, sofern bevorzugterweise keine SMD Komponenten zur Verfügung stehen.

Eine Signalbahn bzw. Signalleitung kann grundsätzlich in unterschiedlicher Tiefe in die Distanzhülse eingebracht sein. Dadurch kann eine zumindest abschnittsweise sehr dünnwandige Distanzhülse realisiert werden. Weiterhin können auch eine Vielzahl von Signalbahnen bzw. Signalleitungen vorgesehen sein, welche in unterschiedlichen Tiefen in die Distanzhülse eingebracht sind. Dies gilt sowohl für an der Außenmantelfläche als auch für an der Innenmantelfläche angebrachte Signalbahnen bzw. Signalleitungen.

Ein elektrischer Kontakt und/oder eine Ausleseantenne kann ferner elektrisch leitend mit der Signalleitung bzw. Signalbahn verbunden sein. Insbesondere kann die oben beschriebene Spule elektrisch leitend mit der Signalleitung bzw. Signalbahn verbunden sein. Dies gilt sowohl für Signalleitungen bzw. -bahnen an der Innenmantelfläche (der Distanzhülse) als auch für Signalleitungen bzw. -bahnen an der Außenmantelfläche der Distanzhülse. Wenn eine Vielzahl von Signalleitungen bzw. -bahnen vorgesehen ist, kann eine Signalbahn bzw. -leitung an einer Seite (beispielsweise der Innenseite) unterbrochen sein und an der anderen Seite (beispielsweise der Außenseite) fortgesetzt werden. Dies kann über eine leitende Verbindung in einer radial verlaufenden Bohrung erreicht werden.

Beispielsweise kann an der Innenmantelfläche der Distanzhülse ein elektrischer Kontakt/ eine elektrische Kontaktfläche für einen Sensor oder für ein anderes (elektronisches) Bauelement aufgebracht sein, welche/r bevorzugt elektrisch leitend mit einer an der Innenmantelfläche aufgebrachten Signalleitung bzw. Signalbahn verbunden ist. Diesbezüglich kann an der Innenmantelfläche der Distanzhülse der elektrische Kontakt/ eine elektrische Kontaktfläche oder mehrere elektrische Kontakte/elektrische Kontaktflächen für die Spule aufgebracht sein, welche/r bevorzugt elektrisch leitend mit einer an der Innenmantelfläche aufgebrachten Signalleitung bzw. Signalbahn verbunden ist. Diesbezüglich können die elektrischen Kontakte/elektrischen Kontaktflächen auch entfallen, wenn die Spule zusammen mit der/den Signalleitung(en) bzw. Signalbahn(en) in der Distanzhülse eingebracht ist.

Ferner ist es denkbar, dass an der Innenmantelfläche eine Ausleseantenne vorgesehen ist, welche bevorzugt elektrisch leitend mit einer an der Innenmantelfläche aufgebrachten Signalleitung bzw. Signalbahn verbunden ist. Dies kann auch derart realisiert werden, dass die Signalleitung bzw. Signalbahn an der Innenmantelfläche derart angeordnet bzw. ausgebildet ist, dass die Signalleitung bzw. Signalbahn selbst die Ausleseantenne bildet. Eine solche Ausleseantenne kann beispielsweise für das Lesen bzw. Beschreiben eines RFID-Chips verwendet werden.

Außerdem kann auch an der Außenmantelfläche der Distanzhülse und insbesondere des Instrumenten-Verlängerungsschafts ein elektrischer Kontakt/ eine elektrische Kontaktfläche für einen Sensor oder für ein anderes Bauelement aufgebracht sein, welche/r bevorzugt elektrisch leitend mit einer an der Außenmantelfläche aufgebrachten Signalleitung bzw. Signalbahn verbunden ist. Die außen angebrachten elektrischen Kontakte bzw. Kontaktflächen können zur Anbindung von außenliegenden Sensoren, (elektronischen) Bauelementen, (Auslese-) Antennen, etc. genutzt werden. Ebenfalls können sie zur Anbindung von in dem Handstück integrierten Bauelementen oder zur Stromversorgung über das Handstück vorgesehen sein.

Außerdem ist es vorteilhaft, wenn die Distanzhülse und/oder der Instrumenten-Verlängerungsschaft aus einer Vielzahl von (zumindest zwei, bevorzugt drei oder mehr) ineinander gesetzten Distanzhülsen besteht. In anderen Worten sollen bevorzugt mehrere Distanzhülsen/Schäfte in mehreren Schichten angeordnet werden. Dadurch lassen sich noch mehr Funktionen in die Distanzhülse/den Schaft integrieren und der Bauraum wird maximal ausgenutzt.

Bevorzugt sind in dem medizinischen Instrument das Wälz-/Kugellager und die Distanzhülse aneinander axial angrenzend angeordnet, derart, dass die zumindest eine Signalleitung bzw. Signalbahn des Wälzlagers mit der zumindest einen Signalleitung bzw. Signalbahn der Distanzhülse über eine Steckverbindung verbunden/ konnektiert ist, so dass das medizinische Instrument zur (multidirektionalen) Signalweiterleitung bzw. Signalübertragung zwischen dem Kugel-/Wälzlager und der Distanzhülse eingerichtet ist.

Somit können in dem medizinischen Instrument/ dem Bohr-/Fräshandstück elektrische Signale über das Kugel-/Wälzlager bzw. dessen Außenring(e) und die Distanzhülse bzw. über eine Vielzahl von Kugel-/Wälzlagern und eine Vielzahl von Distanzhülsen von einem distalen Bereich zu einem proximalen Bereich des medizinischen Instruments und umgekehrt, d.h. in Axialrichtung des medizinischen Instruments, weitergeleitet und übertragen werden.

Dadurch, dass das Kugel-/Wälzlager mit integrierten Signalleitungen und Distanzhülsen mit integrierten Signalleitungen bereitgestellt werden, und die Signalleitungen der Wälzlager mit den Signalleitungen der Distanzhülsen über eine Steckverbindung verbindbar sind, können elektrische Signale sowohl durch diese Bauelemente hindurch als auch zwischen diesen Bauelementen übertragen werden.

Das Wälzlager erlaubt bevorzugt eine Signalübertragung von distal (weg vom Anwender) nach proximal (hin zum Anwender) und umgekehrt, d.h. in Axialrichtung des medizinischen Instruments bzw. des Wälzlagers.

Die Distanzhülse und/oder der Instrumenten-Verlängerungsschaft erlaubt bevorzugt eine Signalübertragung sowohl von distal nach proximal und umgekehrt, d.h. in Axialrichtung des medizinischen Instruments bzw. der Distanzhülse, als auch von innen nach außen und umgekehrt, d.h. in Radialrichtung des medizinischen Instruments bzw. der Distanzhülse.

Insgesamt wird somit eine multidirektionale Signalweiterleitung/ -übertragung in dem medizinischen Instrument/ Handstück (Fräshandstück) bereitgestellt, welche durch das Wälzlager und die Distanzhülsen mit integrierten Signalleitungen/ -bahnen ermöglicht wird.

Es werden in dem medizinischen Instrument gemäß der vorliegenden Offenbarung neue/ erweiterte Funktionen/ Funktionalitäten realisiert, ohne dass der Außendurchmesser bzw. die Außenabmessungen des Instrumenten-Verlängerungsschafts des chirurgischen Instruments/Handstücks vergrößert wird/ werden. Vorliegend kann somit eine miniaturisierte Signalweiterleitung, eine einfache Montage, erweiterte/ neue Platzierungsmöglichkeiten von Signalgebern, Antennen bzw. Sensoren, eine Realisierung von komplexen Schaltungen auf kleinstem Bauraum, und eine geeignete Integration in bestehende Bauelemente bereitgestellt werden.

Mit anderen Worten betrifft die Erfindung einen oder mehrere der folgenden Vorteile/Eigenschaften:
- eine autonome Stromversorgung durch das Handstück selbst,
- eine entkoppelte Stromversorgung von Steuergerät oder nächst im System folgender Steuerungskomponenten,
- eine Integration in den bestehenden Aufbau heutiger Bohr-/Fräshandstücke,
- erleichterte Montage,
- leichterer Anschluss,
- keine komplizierte Anbindung mehrerer Signalgeber bei der Verwendung als Bus-System, sowie
- kein zusätzlicher Kanal für isolierte Litzen, der den Außendurchmesser des Schafts vergrößern würde.

Mit noch anderen Worten betrifft die Erfindung eine Verwendung von Kugellagern gemäß vorstehender Konstruktion als Stromgeneratoren zum Erzeugen/Harvesten (Ernten) von Energie, um elektronische Bauteile wie Sensorik in Vorrichtungen autonom und somit losgekoppelt vom Steuergerät oder nächst im System folgender Steuerungskomponente vorzugsweise ohne aufwändige Zuführung von Energie durch die gesamte Vorrichtung zu betreiben.

Außerdem können die Distanzhülsen (und/oder Instrumenten-Verlängerungsschäfte) elektronische Leitungen aufnehmen, um auch elektronische Bauteile zu beherbergen.

In dieser Variante kann die Distanzhülse eine weitere tragende Rolle einnehmen. Sie kann hierbei als Spule dienen, um Energie zu erzeugen/harvesten. Hierfür können die Kugellager mit Käfig ausgeführt sein. Der Kugelkäfig kann in einer sehr langen Bauform ausgeführt sein. Zum Beispiel werden auf einen mittleren Bereich des Kugelkäfigs Nord/Süd Permanentmagnete aufgebracht, um in Kombination mit der Spule in der Distanzhülse Energie erzeugen/harvesten zu können. Diese kann über die gesamte Länge als auch partiell, einlagig oder mehrlagig ausgelegt werden. Zum Beispiel ist die Spule mäanderförmig ausgebildet. Es ist jedoch auch möglich eine spiralförmig angeordnete Spule einzusetzen. Weiterhin ist es auch denkbar, die Spule mehrlagig auszugestalten, um einen höheren Energieeintrag in die Spule erreichen zu können.

Durch Stecken der Werkzeuge in den Instrumenten-Verlängerungsschaft und Inbetriebnahme des Motors, kann die gesamte innere Baugruppe, zum Beispiel das hierin beschriebene Bauteil (Kugelkäfig), in (Dreh-) Bewegung gebracht werden. Dadurch wird elektrische Energie über die Spule erzeugt und kann für elektronische Schaltungen genutzt werden. Diese Baugruppe kann je nach Länge des Instrumenten-Verlängerungsschafts auch mehrfach in den Instrumenten-Verlängerungsschaft mit eingebracht werden und somit mehrere Spulen zum Erzeugen/Harvesten genutzt werden. Jegliche rotierende Motoren einschließlich Handantrieb können zum Erzeugen/Harvesten geeignet sein.

Ein oder mehrere Ausführungsformen können eine Umsetzung als autonome Baugruppe betreffen. Somit können z.B. Aufsätze bereitgestellt werden, welche vom eigentlichen Antrieb abgekoppelt werden können. Wird ein identischer oder ähnlicher Aufbau gewählt, können auch diese Aufsätze autonom mit Energie versorgt und somit elektronische Bauteile betrieben werden. Dies ist ein weiterer Schritt hin zur Digitalisierung der Produkte. Diese Idee kann auf jeglichen nicht stromversorgenden bzw. nicht stromversorgten Aufsätzen, welche eine Rotationsbewegung beinhalten, skaliert werden.

Mit anderen Worten können in einer Variante Permanentmagnete auf einem Werkzeug oder auf der dahinterliegenden, fest verbauten Antriebswelle vorgesehen sein. Somit könnte auf den Kugelkäfig als Träger für die Permanentmagnete verzichtet werden. Stattdessen würde das Werkzeug die Permanentmagnete tragen. Ein weiterer Vorteil dieser Lösung ist, dass gegenüber dem Kugelkäfig mit dem Werkzeug selbst höhere Rotationsgeschwindigkeiten erreichbar sind und somit der Energieeintrag ebenso höher ist. Um keine Permanentmagnete auf den Werkzeugen aufbringen zu müssen, kann ein Magnetisieren der Werkzeuge ebenso zielführend sein. Dies kann für Niedrigenergieanwendungen vorteilhaft sein.

Mit anderen Worten kann in einer Variante anstelle des gemeinsamen Kugelkäfigs, ein gemeinsamer Innenring ausgebildet sein, der wiederum zum Harvesten mit Permanentmagneten bestückt ist. Dies beinhaltet ebenfalls den Vorteil der höheren Drehzahl und zusätzlich den Vorteil, dass nicht jedes Werkzeug mit Permanentmagneten ausgestattet werden muss. Zusätzlich können in dieser Variante wesentlich größere Permanentmagnete aufgebracht werden um noch mehr Energie zu erzeugen/harvesten.

Somit kann die vorliegende Erfindung mindestens eines der folgenden Eigenschaften aufweisen: Integration von Baugruppen zum Harvesting von Energie in Produkten, Autonome Energieversorgung von elektronischen Bauteilen, Integration von Sensorik und Energieversorgung, Digitalisierung von Vorrichtungen und Kopplung des Erzeugens/Harvestings mit Energie- und Datenspeicher- als auch Kommunikationsmodulen, wie Bluetooth Low Energy, BLE, drahtloses lokales Netzwerk, WLAN, usw., dadurch sind der Nutzbarkeit keine Grenzen gesetzt und dies ist ideal für echte drahtlose Anwendungen (Sensorik im distalen Bereich von Handstücken und Aufsätzen).

Folgende Vorteile können zumindest teilweise erreicht werden:
- Komplexer Aufbau mit Energieversorgung durch Instrumente hindurch kann vermieden werden,
- Autonome Energieversorgung von elektronischen Bauteilen,
- Integration von Sensorik und Energieversorgung der Sensorik ist nun möglich,
- Unabhängige Energieversorgung von Aufsätzen, welche ohne elektrische Verbindung betrieben werden,
- Digitalisierungsfähigkeit von Produkten/Instrumenten.
- Elektronische Bauteile können autonom vom Steuergerät oder nächst im System folgender Steuerungskomponenten versorgt und somit kabellos betrieben werden.

Es ist dem Fachmann klar, dass die hierin dargelegten Erklärungen unter Verwendung von Hardwareschaltungen, Softwaremitteln oder einer Kombination davon implementiert sein/werden können. Die Softwaremittel können im Zusammenhang stehen mit programmierten Mikroprozessoren oder einem allgemeinen Computer, einer ASIC (Application Specific Integrated Circuit) und/oder DSPs (Digital Signal Processors).

Beispielsweise kann das medizinische Instrument teilweise als ein Computer, eine Logikschaltung, ein Field Progammable Gate Array (FPGA), ein Prozessor beispielsweise mit einem Mikroprozessor, einem Mikrocontroller (µC) oder einem Vektorprozessor mit einem Kern oder einer Central Processing Unit (CPU), eine Floating Point Unit (FPU), eine Numeric Processing Unit (NPU), eine Arithmetic Logical Unit (ALU), ein Koprozessor (zusätzlicher Mikroprozessor zur Unterstützung eines Hauptprozessors (CPU)), eine General Purpose Computation on Graphics Processing Unit (GPGPU), ein Parallelrechner (zum gleichzeitigen Ausführen, unter anderem auf mehreren Hauptprozessoren und/oder Grafikprozessoren, von Rechenoperationen) oder ein DSP realisiert sein.

### Kurzbeschreibung der Figuren

Die Offenbarung wird nachfolgend mit Hilfe von Zeichnungen erläutert. Es zeigen:
- Figur 1: eine schematische Darstellung eines medizinischen Instruments/Handstücks mit Instrumenten-Verlängerungsschaft;
- Figur 2: eine schematische Darstellung des Instrumenten-Verlängerungsschafts;
- Figur 3: eine schematische Darstellung eines Kugellagers;
- Figur 4: eine schematische Darstellung eines Bauteils als Rohr/Kugelkäfig mit Permanentmagneten gemäß einem ersten bevorzugten Ausführungsbeispiel der Offenbarung;
- Figur 5: eine schematische Darstellung eines zwischen Kugellagern positionierten Bauteils/Kugelkäfigs mit Permanentmagneten;
- Figur 6: eine schematische Darstellung eines Teils eines Instrumenten-Verlängerungsschafts mit integrierter Spule;
- Figur 7: eine schematische Darstellung als Längsschnitt eines Teils der Distanzhülse mit integrierter Spule;
- Figur 8: eine schematische Teildarstellung eines in einem Instrumenten-Verlängerungsschaft insbesondere einer Distanzhülse mit Spule angeordneten Bauteils (Kugelkäfigs);
- Figur 9: eine schematische offene Darstellung eines in einer Distanzhülse mit Spule angeordneten Bauteils (Kugelkäfigs);
- Figur 10: eine schematische Darstellung mit Handstücken und unterschiedlich vielen Distanzhülsen (innerhalb von Instrumenten-Verlängerungsschäften unterschiedlicher Länge);
- Figur 11: eine schematische Darstellung eines Kugellagers mit Signalleitungen;
- Figur 12: eine schematische Darstellung einer Distanzhülse mit Signalleitungen;
- Figur 13: eine schematische Darstellung unterschiedlicher Aufsätze als Adapter;
- Figur 14: eine schematische Darstellung eines Bauteils als Werkzeug mit Permanentmagneten gemäß einem zweiten bevorzugten Ausführungsbeispiel der Offenbarung; und
- Figur 15: eine schematische Darstellung eines Bauteils als Innenring eines Kugellagers mit Permanentmagneten gemäß einem dritten bevorzugten Ausführungsbeispiel der Offenbarung.

Die Figuren sind lediglich schematischer Natur und dienen ausschließlich dem Verständnis der Erfindung. Die gleichen Elemente sind mit denselben Bezugszeichen versehen. Die Merkmale der einzelnen Ausführungsformen können untereinander ausgetauscht werden.

Darüber hinaus können hier räumlich relative Begriffe, wie etwa "darunter befindlich", "unter", "untere(r)"/"unteres", "darüber befindlich", "obere(r)"/"oberes", "links", "linke(r)/linkes", "rechts", "rechte(r)/rechtes" und dergleichen, zur einfachen Beschreibung der Beziehung eines Elements oder einer Struktur zu einem oder mehreren anderen Elementen oder Strukturen verwendet werden, die in den Figuren dargestellt sind. Die räumlich relativen Begriffe sollen zusätzlich zu der in den Figuren dargestellten Orientierung andere Orientierungen des in Gebrauch oder in Betrieb befindlichen Bauelements umfassen. Das Bauelement kann anders ausgerichtet werden (um 90 Grad gedreht oder in einer anderen Orientierung), und die räumlich relativen Deskriptoren, die hier verwendet werden, können ebenso entsprechend interpretiert werden.

### Figurenbeschreibung

Das medizinische Instrument, der Instrumenten-Verlängerungsschaft, die Distanzhülse und die Kugellager samt dieses verbindenden Bauteils/Kugelkäfigs werden nun anhand von mehreren bevorzugten Ausführungsbeispielen beschrieben.

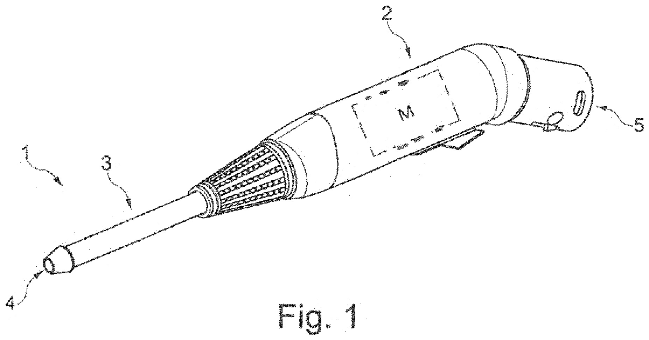

Figur 1 zeigt eine schematische Darstellung eines medizinischen Instruments 1 mit einem Hand-/Griffstück 2 und einem Instrumenten-Verlängerungsschaft (oder einfach Instrumentenschaft) 3, der ein distales Ende aufweist. Der Instrumenten-Verlängerungsschaft 3 hat am distalen Ende eine Aufnahme 4 für ein Werkzeug 17 (Bohr-/Fräswerkzeug), die/das über eine nicht weiter gezeigte Antriebswelle/Stange innerhalb des Instrumenten-Verlängerungsschafts 3 mit einem nicht weiter dargestellten Antrieb (Motor) innerhalb des Handstücks 2 gekoppelt ist, um ein Drehmoment vom Antrieb auf das Werkzeug 17 zu übertragen. Diese Konstruktion zählt zum allgemeinen Stand der Technik der Anmelderin selbst und bedarf daher an dieser Stelle keiner weiteren Ausführungen.

Ferner weist das Handstück 2 einen proximalen Anschluss für eine Energieversorgung, zum Beispiel einen Stromanschluss 5, auf, über den der Antrieb (Motor) innerhalb des Handstücks 2 mit Energie beaufschlagbar ist. Dabei sei an dieser Stelle darauf hingewiesen, dass der gezeigte proximale Anschluss auch eine Schnittstelle für eine Batterie sein kann.

Wie des Weiteren aus der Fig. 1 zu entnehmen ist, existiert zwischen dem Handstück 2 und dem Instrumenten-Verlängerungsschaft 3 eine manuell betätigbare Kupplung 2a zum wahlweisen mechanischen und ggf. elektrischen Anschließen des Instrumenten-Verlängerungsschafts 3 an das Handstück 2. Es ist aber auch möglich, dass der Instrumenten-Verlängerungsschaft 3 fester Bestandteil des Handstücks 2 ist und daher nicht demontiert werden kann.

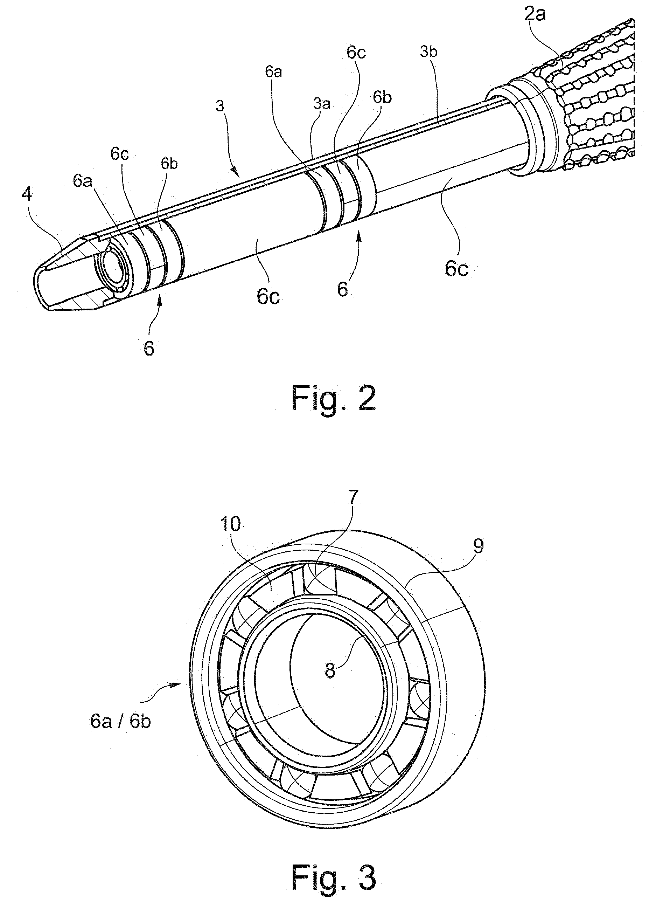

Figur 2 zeigt eine schematische, längs aufgebrochene Darstellung des Instrumenten-Verlängerungsschafts 3.

Der Instrumenten-Verlängerungsschaft 3 weist in diesem Ausführungsbeispiel einen radialen Außenmantel 3a des Verlängerungsschafts 3 auf, an dessen distalem Endabschnitt die Werkzeugaufnahme 4 ausgebildet oder fixiert ist. An der Innenseite des Außenmantels 3a des Verlängerungsschafts 3 ist bevorzugt ein (elektrischer) innerer Isolationsmantel 3b des Verlängerungsschaftes 3 eingesetzt. Es ist aber auch möglich, dass die innen liegenden Komponenten ohne inneren Isolationsmantel 3b des Verlängerungsschafts 3 in den Instrumenten-Verlängerungsschaft 3 eingesetzt werden.

Längs des Instrumenten-Verlängerungsschafts 3 sind mehrere Kugellagerpaare 6 angeordnet, die jeweils in Axialrichtung des Instrumenten-Verlängerungsschafts 3 zueinander beabstandet sind, sodass die nicht weiter dargestellte Antriebswelle bzw. ein mit der Welle in Verbindung gebrachtes Werkzeug 17 entsprechend in dem Instrumenten-Verlängerungsschaft 3 drehbar gelagert werden kann bzw. eine Rotationskraft (Drehmoment) von dem Motor des medizinischen Instruments 1 über die Welle auf das Werkzeug 17 übertragen werden kann.

Jedes Kugellagerpaar 6, vorzugsweise zumindest das distal gelegene Kugellagerpaar 6, hat zwei ausgewählte, axial beabstandete Einzelkugellager 6a, 6b und eine Distanzhülse 6c axial zwischen den zwei ausgewählten Einzelkugellagern 6a, 6b. Jedes so definierte ausgewählte Kugellagerpaar 6 der vorstehenden Konzeption ist dabei in den Instrumenten-Verlängerungsschaft 3, vorzugsweise in den inneren Isolationsmantel des Verlängerungsschafts 3b, axial unbeweglich eingesetzt.

Figur 3 zeigt eine schematische Darstellung eines solchen Einzelkugellagers 6a, 6b. Jedes Einzelkugellager 6a, 6b des gleichen Kugellagerpaares 6 weist bevorzugt Kugeln 7 als Wälzkörper auf, wobei natürlich auch andere Wälzkörperformen vorgesehen sein können. Das Einzelkugellager (nachfolgend einfach als Kugellager bezeichnet) 6a, 6b hat ferner einen Innenring 8 und einen Außenring 9, zwischen denen die Kugeln 7 gelagert sind. Die Kugeln 7 sind durch einen Kugelkäfig 10 in Umfangsrichtung auf Abstand gehalten.

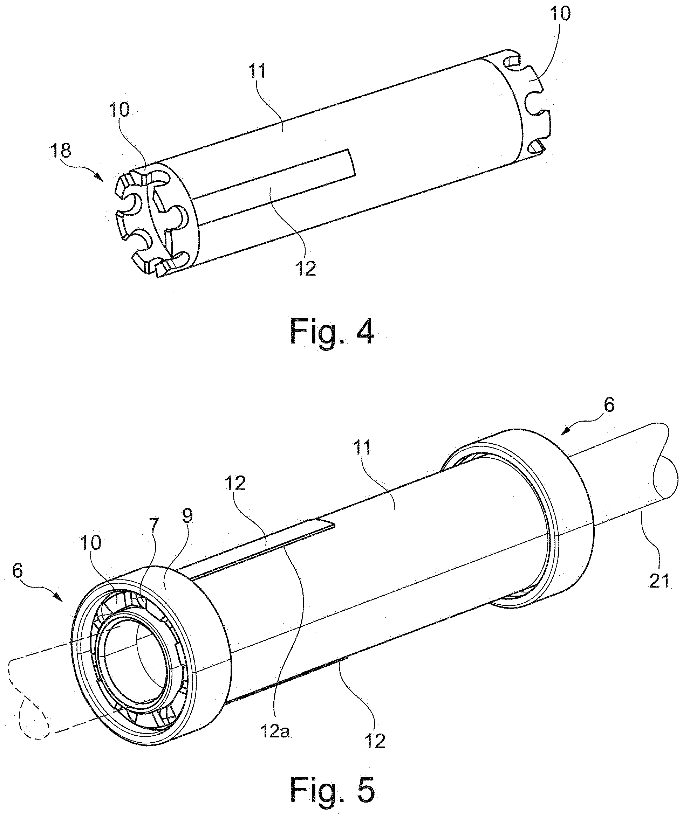

Der Kugelkäfig 10 jedes Kugellagers 6a, 6b eines Kugellagerpaares 6 ist in der Fig. 4 schematisch gezeigt.

Demzufolge bildet jeder Kugelkäfig 10 umfangsbeabstandete axial sich erstreckende Vorsprünge/Zähne, zwischen denen sich taschenförmige Kugelaufnahmen ausformen, in welche die Kugeln 7 einzeln eingesetzt sind. Die Kugelkäfige 10 der paarweise zusammengefassten Kugellager 6a, 6b sind in diesem bevorzugten Ausführungsbeispiel über einen zylindrischen Abschnitt 11 miteinander quasi zu einem einzigen, gemeinsamen Käfigbauteil oder Rohr 18 fest verbunden.

Das in Figur 4 gezeigte Rohr 18 weist demnach zumindest den zylindrischen Abschnitt 11 auf, an dessen axiale Stirnseiten die Kugelkäfige 10 der beiden axial beabstandeten Kugellager 6a, 6b des einen Kugellagerpaares 6 fest angeordnet sind. Der zylindrische Abschnitt 11 weist zudem an seinem Außenumfang nahe dem einen Kugelkäfig 10 zumindest einen, vorzugsweise mehrere umfangsbeabstandete Permanentmagnete 12 auf. Die Kugelkäfige 10 können dabei zusammen mit dem zylindrischen Abschnitt 11 einstückig ausgebildet oder als separate Bauteile mit diesem fest verbunden sein. Jeder, vorzugsweise streifenförmige, Permanentmagnet 12 ragt ferner ausgehend von nur dem einen Kugelkäfig 10 in Axialrichtung in etwa bis zur axialen Mitte des zylindrischen Abschnitts 11.

An dieser Stelle sei zum besseren Verständnis darauf hingewiesen, dass zumindest die axiale Dimensionierung des Rohrs 18 gemäß der Fig. 4 ggf. unrealistisch ist und nur zu Darstellungszwecken dient. Vielmehr kann insbesondere der zylindrische Abschnitt 11 zwischen zwei beliebig ausgewählten, zu einem Paar gekoppelten Kugellagern 6a, 6b in der Realität auch deutlich kürzer oder länger sein, wie dies in Fig. 2 durch die unterschiedlich langen Distanzhülsen 6c dargestellt ist. Insbesondere sei in diesem Zusammenhang darauf hingewiesen, dass gemäß der Offenbarung auch das in Fig. 2 am distalen Instrumenten-Verlängerungsschaft 3 angeordnete Kugellager 6b mit dem in Richtung proximal daran anschließenden Kugellager 6a zu einem Kugellagerpaar 6 gekoppelt werden können. D.h. unter dem Kugellagerpaar 6 wird allgemein gemäß der Offenbarung ein Paar axial nebeneinanderliegender Kugellager verstanden, so dass das Rohr 18 und insbesondere der zylindrische Abschnitt 11, wie vorstehend erwähnt, in seiner axialen Erstreckung variieren kann. Somit kann auch die in Fig. 2 längere Distanzhülse 6c für die Spule 13 genutzt werden.

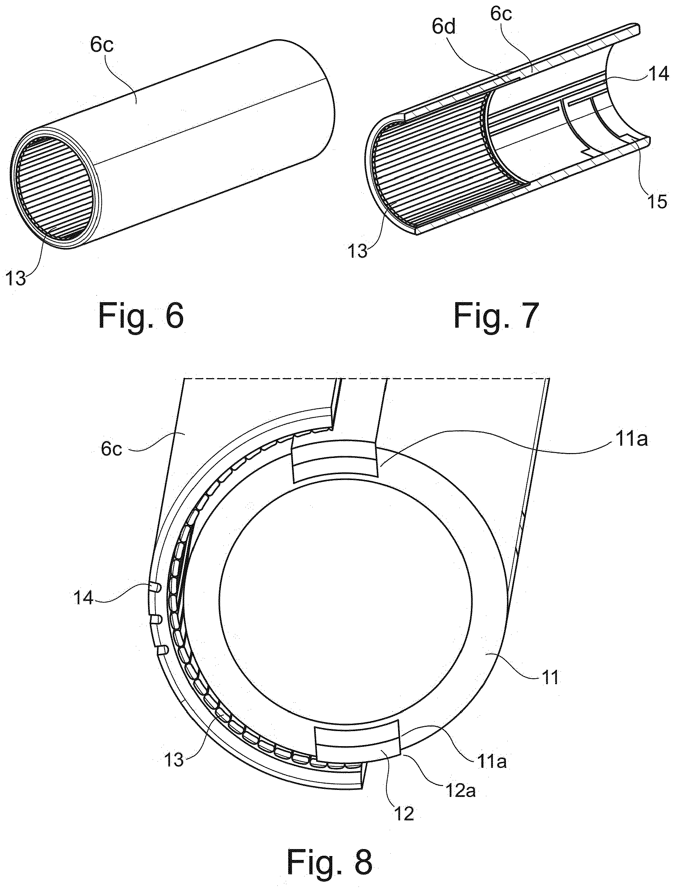

Figur 5 zeigt eine schematische Darstellung des zwischen den Kugellagern 6a, 6b des einen Kugellagerpaares 6 positionierten Rohrs 18 mit Permanentmagneten 12. Demzufolge greifen die axialen Vorsprünge/Zähne jedes Kugelkäfigs 10 zwischen die Kugeln 7 der beiden Kugellager 6a, 6b, sodass die Kugelkäfige 10 zusammen mit den Kugeln 7 entlang des Umfangs zwischen dem Innenring 8 und dem Außenring 9 drehbar sind. Ferner kann das Rohr 18 so zwischen den Kugellagern 6a, 6b des einen Kugellagerpaares 6 angeordnet sein, dass es nicht axial verschiebbar ist. Schließlich ist aus der Fig. 5 zu entnehmen, dass vorzugsweise zwei Permanentmagnete 12 vorgesehen sind, die in diesem Fall diametral zueinander am zylindrischen Abschnitt 11 des Rohrs 18 positioniert sind. Die Permanentmagnete 12 sind dabei in eine Aussparung 11a an der äußeren Mantelseite des zylindrischen Abschnitts 11 eingesetzt (wie dies in Fig. 6 gezeigt ist), derart, dass die Permanentmagnete 12 über die Mantelseite vorragen und so einen radial vorragenden Absatz 12a um den Permanentmagneten 12 herum ausbilden. Die Permanentmagnete 12 können aber auch bündig mit der äußeren Mantelseite des zylindrischen Abschnitts 11 abschließen.

Figur 6 und 7 zeigen eine schematische Darstellung eines Teils der Distanzhülse 6c mit integrierter Spule 13.

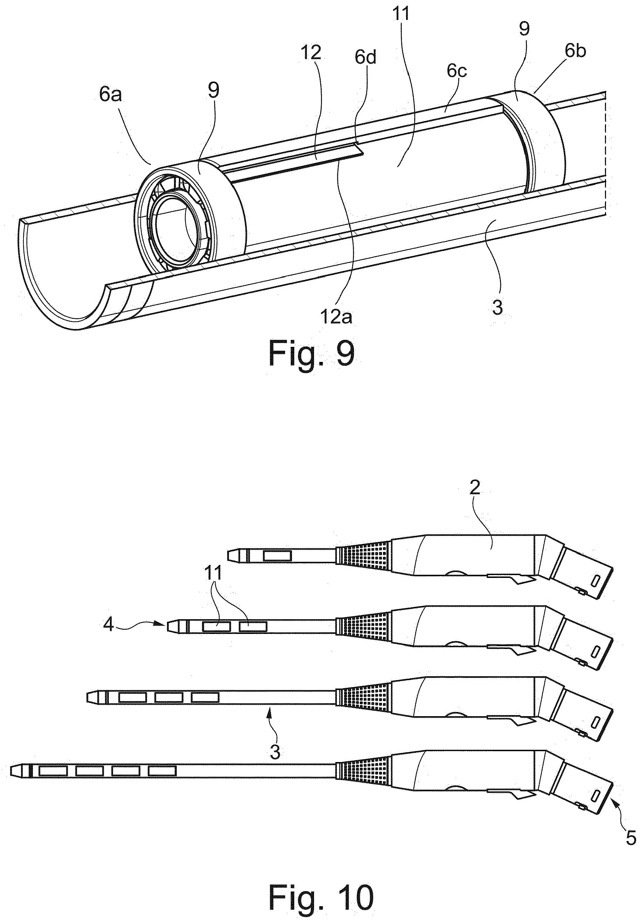

Die (längsgeschlitze) Distanzhülse 6c ist im vorliegenden bevorzugten Ausführungsbeispiel separat zum Instrumenten-Verlängerungsschaft 3 ausgebildet und hält die beiden Kugellager 6a, 6b auf axiale Distanz zueinander. Hierfür liegt die Distanzhülse 6c bevorzugt an den zueinander gerichteten Stirnseiten der Außenringe 9 der beiden Kugellager 6a, 6b des gleichen Kugellagerpaares 6 an und umgibt somit radial außenseitig den zylindrischen Abschnitt 11 des Rohrs 18 (siehe insbesondere Fig. 9).

Die Distanzhülse 6c ist ausgehend von (nur) einer Stirnseite über eine axiale Länge bis in etwa deren axiale Mitte mit einer radialen Ausdrehung/Ausbuchtung 6d an deren innerer Mantelseite versehen, an/in welche die Spule 13 eingesetzt ist, welche mäanderförmig oder spiralförmig ausgebildet ist. Die Spule 13 kann somit zumindest eine Hälfte des Innenumfangs der Distanzhülse 6c ausmachen. Ferner kann die Spule entlang des Innendurchmesserumfangs des Instrumenten-Verlängerungsschafts 3 angeordnet sein. Die radiale Ausbuchtung 6d ist derart dimensioniert, dass (wie insbesondere in Fig. 9 gezeigt) die ggf. radial vorragenden Permanentmagnete 12 darin kontaktlos aufgenommen werden können.

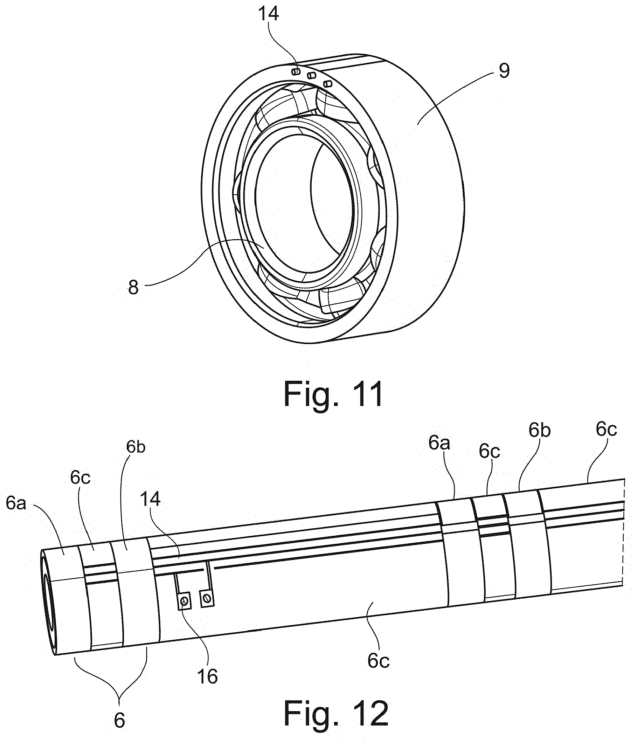

Ferner sind an der radial inneren Mantelseite der Distanzhülse 6c in einem an die Spule 13 axial angrenzenden Axialabschnitt eine Anzahl von Signalleitungen 14 und damit verbundene innere Kontakte 15 angeordnet/ausgebildet. Insbesondere sind die Signalleitungen 14 weitgehend in Längsrichtung der Distanzhülse 6c so angeordnet, dass sie an einer axialen Stelle mit der Spule 13 (elektrisch) verbunden sind. Die inneren Kontakte 15 sind dabei bevorzugt über radiale Durchgangsbohrungen/Durchgangsleitungen sowie über radial äußere Kontakte 16 mit radial äußeren, axial sich erstreckenden Signalleitungen 14 der Distanzhülse 6c in (elektrischem) Kontakt. (siehe insbesondere auch Fig. 12).

Figur 8 zeigt eine schematische Teildarstellung des in der Distanzhülse 6c mit Spule 13 angeordneten Rohrs 18. Hierbei sieht man den zylindrischen Abschnitt 11, der die vorstehend genannten Aussparungen 11a aufweist, die die Permanentmagnete 12 beinhalten, die jeweils einen in Radialrichtung ausgerichteten Nordpol und Südpol aufweisen und jeweils durch unterschiedliche Schichten gezeigt sind. Die Nord- und Südpole der jeweiligen Permanentmagnete 12 liegen sich an dem zylindrischen Abschnitt 11 in Radialrichtung gegenüber, wobei die entsprechenden Nordpole oder die entsprechenden Südpole nach radial außen zeigen. Die Anordnung der Nord- und Südpole erfolgt bei zwei Permanentmagneten 12 folgendermaßen:
Wenn beim in Fig. 8 oberen Permanentmagneten Norden radial außen ist, dann ist beim unteren Permanentmagneten Süden radial außen. Auf der radialen Innenseite der Distanzhülse 6c stehen sich somit Norden und Süden gegenüber. Somit können sich die Feldlinien entsprechend dem Prinzip eines Stromgenerators ausbilden.

An einem Außenumfang der Distanzhülse 6c verlaufen die Signalleitungen 14, die in Figur 7 an einer Innenseite der Distanzhülse 6c in Längsrichtung ebenfalls angeordnet sind, axial weiter, wie in Figur 8 zu sehen ist.

Figur 9 zeigt hierfür eine schematische offene Darstellung des in die Distanzhülse 6c mit Spule 13 angeordneten Rohrs 18 als Bauteil (Rotor des Generators). Demzufolge ist das ausgewählte Kugellagerpaar 6 samt Rohr 18 zu einer Art Einheit/Patrone zusammengefasst, die geschlossen in den Instrumenten-Verlängerungsschaft 3 eingesetzt ist. An dieser Stelle sei darauf hingewiesen, dass der Instrumenten-Verlängerungsschaft nur ein bevorzugter Einbauort des Kugellagerpaares ist, wobei dieses auch an anderen Orten, beispielsweise innerhalb eines Gehäuses des Handstücks 2 angeordnet sein kann.

Des Weiteren können Instrumenten-Verlängerungsschäfte 3 unterschiedlicher Länge vorgesehen sein. In Figur 10 ist eine schematische Darstellung von medizinischen Handstücken 2 mit unterschiedlich langen instrumenten-Verlängerungsschäften 3 gezeigt. Innerhalb der unterschiedlichen Verlängerungsschäfte kann eine unterschiedliche Anzahl an längsbeabstandeten Distanzstücken 6c ebenfalls mit unterschiedlicher Länge angeordnet werden, die das jeweilige Bauteil (Kugelkäfig) radial umgeben.

Figur 11 zeigt eine schematische Darstellung eines Kugellagers 6a/6b eines ausgewählten Kugellagerpaars 6 mit Signalleitungen 14, wie vorstehend beschrieben, die in den radial äußeren Außenring 9 eingesetzt sind. Wie aus der Fig. 11 gut zu entnehmen ist, bilden die Signalleitungen 14 zumindest an der einen, vorzugsweise ab beiden Stirnseiten des radial äußeren Außenrings 9 axial vorragende Kontaktstifte, welche bei der Montage eines ausgewählten Kugellagerpaares 6 in entsprechende axiale Buchsen in der Distanzhülse 6c eingreifen und so einen elektrischen Kontaktschluss zwischen den Signalleitungen 14 in der Distanzhülse 6c und den Signalleitungen 14 im radial äußeren Außenring 9 herstellen.

Figur 12 zeigt hierzu eine schematische Darstellung eines Instrumenten-Verlängerungsschafts 3 mit radial äußeren Signalleitungen 14, wie ebenfalls oben beschrieben. Demzufolge erstrecken sich die Signalleitungen 14 ausgehend vom distalen Kugellagerpaar 6 bis in das Handstück 2, wobei die Signalleitungen in den radial äußeren Außenring aller Kugellager und die Signalleitungen aller Distanzhülsen nach dem vorstehenden Buchsen-Stift-Prinzip elektrisch koppeln. Ferner sind in Fig. 12 die vorstehend genannten Kontaktstellen 16 an einer ausgewählten Distanzhülse 6c gezeigt, die über radiale Kontakte mit den radial inneren Kontaktstellen 15 verbunden sind. Auf diese Weise haben die radial inneren Signalleitungen der jeweils ausgewählten Distanzhülse 6c mit deren radial äußeren Signalleitungen elektrische Verbindung.

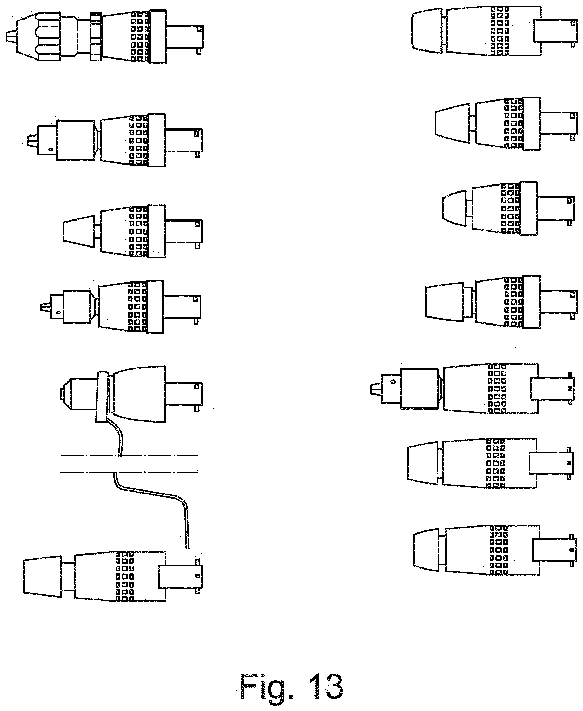

Des Weiteren zeigt Figur 13 eine schematische Darstellung unterschiedlicher Werkzeuge bzw. Aufsätze 17 als Adapter. Von oben nach unten sind auf der linken Seite ein schlüsselloser 3-Backen Bohraufsatz, ein 3-Backen Bohraufsatz (0,5-7,4mm), ein AO klein Bohraufsatz, ein kleiner 3-Backen Bohraufsatz, ein Spickdrahtaufsatz, ein Hudson/Zimmer Fräsaufsatz zu sehen. Von oben nach unten sind auf der rechten Seite ein großer AO Markraum Bohraufsatz, ein kleiner AO Bohraufsatz, ein 6-Kant Bohraufsatz, ein Hudson/Zimmer Bohraufsatz, ein 3-Backen Fräsaufsatz (0,5-7,4mm), ein großer AO Fräsaufsatz und ein Harris Fräsaufsatz zu sehen.

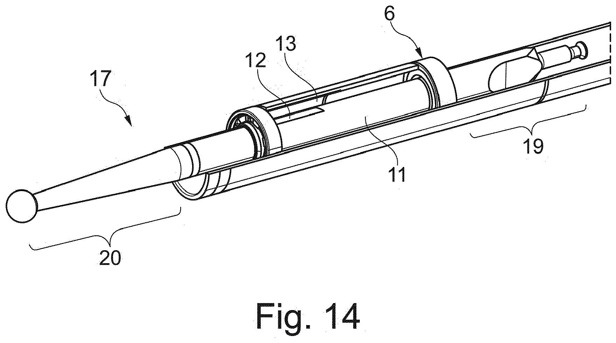

Figur 14 zeigt eine schematische Darstellung eines medizinischen Instruments und insbesondere eines Instrumenten-Verlängerungsschafts 3 gemäß einem zweiten bevorzugten Ausführungsbeispiel der Offenbarung mit einem Bauteil in Form des Werkzeug 17 selbst, d.h. dass in diesem Fall das Rohr 18 gemäß dem ersten bevorzugten Ausführungsbeispiel durch den Schaft des Werkzeugs 17 ersetzt ist. Das Werkzeug 17 weist zu diesem Zweck einen distalen Effektorabschnitt 20 auf, der über einen in den Verlängerungsschaft 3 einsetzbaren zylindrischen Schaftabschnitt 11 mit einem proximalen Kopplungsabschnitt 19 des Werkzeugs 17 verbunden ist, mittels dem das Werkzeug 17 axial- und drehfest mit der (nicht genauer gezeigten) Antriebswelle 21 innerhalb des Instrumenten-Verlängerungsschafts 3 koppelbar ist. Der zylindrische Abschnitt 11 des Werkzeugs 17 weist die Permanentmagnete 12 auf, die zum Induzieren eines Stroms in die Spule 13 der zumindest einen, in Figur 14 im Halbschnitt dargestellten Distanzhülse 6c vorgesehen sind, welche im proximal zur Werkzeugaufnahme 4 unmittelbar sich anschließenden Schaftabschnitt vorgesehen ist und den Werkzeugschaft radial umgibt. Der zylindrische Abschnitt 11 des Werkzeugs 17 kann so zwischen den Kugellagern 6a, 6b des in diesem Fall distalen Kugellagerpaares 6 angeordnet sein, dass insbesondere der vorstehend beschriebene elektrische Induktions-Effekt zwischen dem Werkzeug 17 und der Distanzhülse 6c zum Ernten von elektrischer Energie ausgenutzt wird. Der zylindrische Abschnitt 11 des Werkzeugs 17 schließt nahtlos an den Kopplungsabschnitt 19 auf der einen Axialseite und den Effektorabschnitt 20 auf der anderen Axialseite an. Hierdurch werden die in den zylindrischen Abschnitt 11 des Werkzeugs 17 eigesetzten Permanentmagnete 12 im Uhrzeigersinn oder gegen den Uhrzeigersinn gedreht, wodurch ein veränderliches Magnetfeld während der Drehbewegung des Werkzeugs 17 entsteht, dass einen Strom in der Spule 13 erzeugt. Hierdurch lässt sich auf einfache Weise Energie erzeugen/ernten. Dieses Prinzip gilt für jedes der hierin beschriebenen Bauteile.

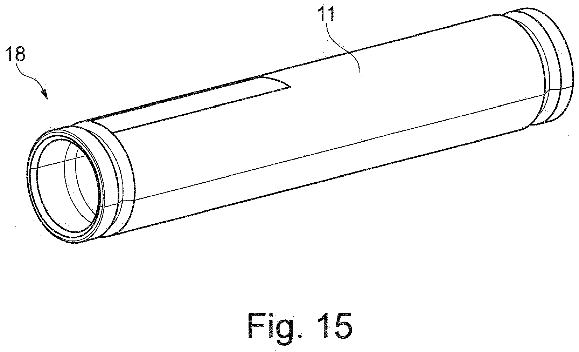

Figur 15 zeigt eine schematische Darstellung eines Innenrings 8 eines Kugellagerpaars 6 gemäß einem weiteren bevorzugten Ausführungsbeispiel der Offenbarung als jenes Bauteil mit Permanentmagneten 12. Hierbei kann der Innenring 8 fest mit der Antriebswelle innerhalb des Instrumenten-Verlängerungsschafts 3 oder des medizinischen Instruments 1, oder dem Werkzeug 17 selbst verbunden sein. Somit kann ein über die Antriebswelle 21 übertragenes Drehmoment vollständig von dem Innenring 8 aufgenommen werden. Hierdurch lässt sich effektiv Energie erzeugen/ernten.

### Bezugszeichenliste

- 1: Medizinische Instrument
- 2: Handstück
- 2a: manuell betätigbare Kupplung
- 3: Instrumenten-Verlängerungsschaft
- 3a: Außenmantel des Verlängerungsschafts
- 3b: innerer Isolationsmantel des Verlängerungsschafts
- 4: Werkzeugaufnahme
- 5: Stromanschluss
- 6: Ausgewähltes Kugellagerpaar
- 6a, 6b: Kugellager des ausgewählten Kugellagerpaars
- 6c: ausgewählte Distanzhülse
- 6d: innerer radialer Rücksprung (innere radiale Aufweitung)
- 7: Kugeln des Kugellagers
- 8: Innenring des Kugellagers
- 9: Außenring des Kugellagers
- 10: Kugelkäfig des Bauteils
- 11: zylindrischer Abschnitt des Bauteils
- 11a: Aussparung
- 12: Permanentmagnete
- 12a: Absatz
- 13: Spule
- 14: Signalleitungen
- 15: innerer Kontakt
- 16: äußerer Kontakt
- 17: Werkzeug
- 18: Bauteil als Rohr
- 19: Kopplungsabschnitt
- 20: Effektorabschnitt
- 21: Antriebswelle

## Patentansprüche

1. Medizinisches, motor- oder handbetriebenes oder betreibbares Instrument (1) mit einer Anzahl von Lagern zur Lagerung eines Schafts oder einer Welle (21) für eine Drehmomentbeaufschlagung eines Werkzeugs (17), von denen wenigstens zwei ausgewählte oder auswählbare Lager (6a, 6b) ein Lagerpaar (6) bilden und einer Distanzhülse (6c), welche die Lager (6a, 6b) des Lagerpaars (6) axial beabstandet, wobei das Instrument weiterhin beinhaltet:
i) ein zumindest teilweise radial innerhalb der Distanzhülse (6c) liegendes, vorzugsweise rohrförmiges Bauteil (11), welches mit zumindest einem der beiden Lager (6a, 6b) des Lagerpaars (6) derart drehgekoppelt oder drehkoppelbar ist, um mit jeweils einem drehenden Teil des zumindest einen Lagers (6a, 6b) des Lagerpaars (6) mitzudrehen oder einen drehenden Teil des zumindest einen Lagers (6a, 6b) des Lagerpaars (6) zu bilden,
ii) zumindest einen Permanentmagneten (12), der an oder in dem Bauteil (11) befestigt oder ausgebildet ist und
iii) eine Spule (13), die an oder in der Distanzhülse (6c) angeordnet ist.

2. Medizinisches Instrument (1) nach Anspruch 1, **dadurch gekennzeichnet, dass** die Lager (6a, 6b) des Lagerpaars (6) Wälzlager, vorzugsweise Kugellager sind und das Bauteil (11) ein gemeinsamer Bestandteil von Kugelkäfigen (10) der beiden Lager (6a, 6b) ist, vorzugsweise ein zylindrischer Abschnitt (11), der die Kugelkäfige (10) der beiden Lager (6a, 6b) des Lagerpaars (6) drehfest miteinander koppelt.

3. Medizinisches Instrument (1) nach Anspruch 1, **dadurch gekennzeichnet, dass** das Bauteil (11) ein Schaftabschnitt des Werkzeugs (17) ist.

4. Medizinisches Instrument (1) nach Anspruch 1, **dadurch gekennzeichnet, dass** die Lager (6a, 6b) des Lagerpaars (6) Wälzlager, vorzugsweise Kugellager sind und das Bauteil (11) ein gemeinsamer Bestandteil von Innenringen (8) der beiden Lager (6a, 6b) ist, vorzugsweise ein zylindrischer Abschnitt (11), der die Innenringe (8) der beiden Lager (6a, 6b) des Lagerpaars (6) drehfest miteinander koppelt.

5. Medizinisches Instrument (1) nach einem der vorstehenden Ansprüche 1 bis 4, **dadurch gekennzeichnet, dass** das medizinische Instrument ein Handinstrument mit einem Hand- oder Griffstück (2) ist.

6. Medizinisches Instrument nach Anspruch 5, **dadurch gekennzeichnet, dass** in dem Handstück (2) ein Motor untergebracht ist, der mit der Welle (21) für eine Drehmomentübertragung auf das Werkzeug (17) drehgekoppelt oder drehkoppelbar ist, wobei die beiden Lager (6a, 6b) sowie die Distanzhülse (6c) in einem Instrumenten-Verlängerungsschaft (3) angeordnet sind, der an das Hand- oder Griffstück angekoppelt oder ankoppelbar ist.

7. Medizinisches Instrument (1) nach Anspruch 6, **gekennzeichnet durch** eine vorzugsweise manuell betätigbare Kupplung (2a) über welche der Verlängerungsschaft (3) mit dem Handstück (2) und auch die im Verlängerungsschaft (3) gelagerte Welle (21) mit dem im Handstück (2) befindlichen Motor koppelbar ist.

8. Medizinisches Instrument (1) nach Anspruch 7, **dadurch gekennzeichnet, dass** der Instrumenten-Verlängerungsschaft (3) über die Kupplung (2a) mechanisch und/ oder elektrisch an das Handstück (2) anschließbar ist.

9. Medizinisches Instrument (1) nach einem der Ansprüche 7 bis 8, **dadurch gekennzeichnet, dass** das Handstück (2) einen distalen Endabschnitt aufweist, an dem die Kupplung (2a) angeordnet ist, wobei an einem distalen Endabschnitt des Instrumenten-Verlängerungsschafts (3) eine Werkzeugaufnahme (4) angeordnet ist.

10. Medizinisches Instrument (1) nach einem der Ansprüche 1 bis 9, **dadurch gekennzeichnet, dass** das Lagerpaar (6) einschließlich zumindest der Distanzhülse (6c) und vorzugsweise des Bauteils (11) eine separate Einheit zur Montage in das medizinische Instrument (1) bildet.

11. Medizinisches Instrument (1) nach einem der vorstehenden Ansprüche 1 bis 9, **dadurch gekennzeichnet, dass** die Distanzhülse (6c) fester Bestandteil des medizinischen Instruments (1) ist.

12. Medizinisches Instrument (1) nach einem der vorstehenden Ansprüche 1 bis 11, **dadurch gekennzeichnet, dass** die Spule (13) mäander- oder spiralförmig sowie, in Radialrichtung der Distanzhülse (6c) gesehen, ein- oder mehrlagig ist.

13. Medizinisches Instrument (1) nach einem der Ansprüche 1 bis 12, **dadurch gekennzeichnet, dass** das Bauteil (11) an dessen äußerer Mantelseite eine Anzahl von Ausnehmungen (11a) hat, in welche die entsprechende Anzahl von Permanentmagneten (12) eingesetzt ist.

14. Medizinisches Instrument (1) nach einem der Ansprüche 1 bis 13, **dadurch gekennzeichnet, dass** zumindest die Distanzhülse (6c) radial innenliegende Signalleitungen (14) hat, die mit der Spule (13) sowie mit radial außenliegenden Signalleitungen (14) elektrisch verbunden sind, welche über elektrische Kupplungen mit Signalleitungen in den Lagern (6a, 6b) gekoppelt sind, um so elektrische Signale ausgehend von der Spule (13) längs des medizinischen Instruments (1) nach proximal zu leiten.

## Claims

1. A medical, motor-operated or hand-operated or operable instrument (1) comprising a number of bearings for supporting a shank or shaft (21) for applying torque to a tool (17), of which at least two selected or selectable bearings (6a, 6b) form a pair of bearings (6), and a distance sleeve (6c) axially spacing the bearings (6a, 6b) of the pair of bearings (6), the instrument further including:
i) a preferably tubular component (11) lying at least partly radially inside the distance sleeve (6c), which component (11) is rotatably coupled or capable of being coupled with at least one of the two bearings (6a, 6b) of the pair of bearings (6) so as to co-rotate with a respective rotating part of the at least one bearing (6a, 6b) of the pair of bearings (6), or to form a rotating part of the at least one bearing (6a, 6b) of the pair of bearings (6), ii) at least one permanent magnet (12) attached to or configured in the component (11), and iii) a coil (13) disposed on or in the distance sleeve (6c).

2. The medical instrument (1) according to claim 1, **characterized in that** the bearings (6a, 6b) of the pair of bearings (6) are roller bearings, preferably ball bearings, and the component (11) is a common part of ball cages (10) of the two bearings (6a, 6b), preferably a cylindrical portion (11), which couples the ball cages (10) of the two bearings (6a, 6b) of the pair of bearings (6) to each other in a rotationally fixed manner.

3. The medical instrument (1) according to claim 1, **characterized in that** the component (11) is a shaft portion of the tool (17).

4. The medical instrument (1) according to claim 1, **characterized in that** the bearings (6a, 6b) of the pair of bearings (6) are roller bearings, preferably ball bearings, and the component (11) is a common part of inner rings (8) of the two bearings (6a, 6b), preferably a cylindrical portion (11), which couples the inner rings (8) of the two bearings (6a, 6b) of the pair of bearings (6) in a rotationally fixed manner.

5. The medical instrument (1) according to one of the preceding claims 1 to 4, **characterized in that** the medical instrument is a hand instrument having a handpiece or gripping section (2).

6. The medical instrument according to claim 5, **characterized in that** the handpiece (2) accommodates a motor which is rotationally coupled or can be rotationally couplable to the shaft (21) for torque transmission to the tool (17), wherein the two bearings (6a, 6b) as well as the distance sleeve (6c) are arranged in an instrument elongation shaft (3) which is coupled or couplable to the handpiece or gripping section.

7. The medical instrument (1) according to claim 6, **characterized by** a preferably manually-operable coupling (2a) via which the elongation shaft (3) is couplable to the handpiece (2) and also the shaft (21) mounted in the elongation shaft (3) is couplable to the motor located in the handpiece (2).

8. The medical instrument (1) according to claim 7, **characterized in that** the instrument elongation shaft (3) can be mechanically and/or electrically connected to the handpiece (2) via the coupling (2a).

9. The medical instrument (1) according to one of claims 7 to 8, **characterized in that** the handpiece (2) has a distal end portion at which the coupling (2a) is arranged, wherein a tool receptacle (4) is arranged at a distal end portion of the instrument elongation shaft (3).

10. The medical instrument (1) according to one of claims 1 to 9, **characterized in that** the pair of bearings (6) including at least the distance sleeve (6c) and preferably the component (11) forms a separate unit for mounting into the medical instrument (1).

11. The medical instrument (1) according to one of the preceding claims 1 to 9, **characterized in that** the distance sleeve (6c) is an integral part of the medical instrument (1).

12. The medical instrument (1) according to one of the preceding claims 1 to 11, **characterized in that** the coil (13) is meander-shaped or spiral-shaped and, viewed in the radial direction of the distance sleeve (6c), is single-layered or multilayered.

13. The medical instrument (1) according to one of claims 1 to 12, **characterized in that** the component (11) has, on its outer jacket surface, a number of recesses (11a) into which the corresponding number of permanent magnets (12) are inserted.

14. The medical instrument (1) according to one of claims 1 to 13, **characterized in that** at least the distance sleeve (6c) has radially inner signal lines (14) electrically connected to the coil (13), and radially outer signal lines (14) coupled via electrical couplings to signal lines in the bearings (6a, 6b) so as to conduct electrical signals from the coil (13) proximally along the medical instrument (1).

## Revendications

1. Instrument médical (1) entraîné ou pouvant être entraîné par un moteur ou la main avec une pluralité de paliers pour le montage d'une tige ou d'un arbre (21) pour une application de couple de rotation d'un outil (17), dont au moins deux paliers sélectionnés ou sélectionnables (6a, 6b) forment une paire de paliers (6), et une douille entretoise (6c), laquelle sépare axialement les paliers (6a, 6b) de la paire de paliers (6), dans lequel l'instrument inclut en outre :
i) un composant (11), de préférence tubulaire, se trouvant au moins en partie radialement à l'intérieur de la douille entretoise (6c) qui est couplé en rotation ou apte à être couplé en rotation avec au moins un des deux paliers (6a, 6b) de la paire de paliers (6) pour tourner avec respectivement une partie rotative de l'au moins un palier (6a, 6b) de la paire de paliers (6) ou pour former une partie rotative de l'au moins un palier (6a, 6b) de la paire de paliers (6),
ii) au moins un aimant permanent (12) qui est fixé ou conçu au niveau du composant (11) ou dans celui-ci et
iii) une bobine (13) qui est disposée au niveau de la douille entretoise (6c) ou dans celle-ci.

2. Instrument médical (1) selon la revendication 1, **caractérisé en ce que** les paliers (6a, 6b) de la paire de paliers (6) sont des paliers à roulement, de préférence des roulements à billes, et le composant (11) est un élément commun de cages à billes (10) des deux paliers (6a, 6b), de préférence une section cylindrique (11) qui couple entre elles de manière fixe en rotation les cages à billes (10) des deux paliers (6a, 6b) de la paire de paliers (6).

3. Instrument médical (1) selon la revendication 1, **caractérisé en ce que** le composant (11) est une section de tige de l'outil (17).

4. Instrument médical (1) selon la revendication 1, **caractérisé en ce que** les paliers (6a, 6b) de la paire de paliers (6) sont des paliers à roulement, de préférence des roulements à billes, et le composant (11) est un élément commun de bagues internes (8) des deux paliers (6a, 6b), de préférence une section cylindrique (11) qui couple entre elles de manière fixe en rotation les bagues internes (8) des deux paliers (6a, 6b) de la paire de paliers (6).

5. Instrument médical (1) selon l'une quelconque des revendications 1 à 4, **caractérisé en ce que** l'instrument médical est un instrument à main avec une pièce à main ou un manche (2).

6. Instrument médical selon la revendication 5, **caractérisé en ce que** dans la pièce à main (2) est logé un moteur qui est couplé en rotation ou apte à être couplé en rotation à l'arbre (21) pour une transmission de couple de rotation à l'outil (17), dans lequel les deux paliers (6a, 6b) ainsi que la douille entretoise (6c) sont disposés dans une tige d'extension d'instrument (3) qui est couplée ou apte à être couplée à la pièce à main ou au manche.

7. Instrument médical (1) selon la revendication 6, **caractérisé par** un couplage (2a) pouvant être actionné de préférence manuellement par l'intermédiaire duquel la tige d'extension (3) est apte à être couplée à la pièce à main (2) et l'arbre (21) logé dans la tige d'extension (3) est également apte à être couplé au moteur se trouvant dans la pièce à main (2).

8. Instrument médical (1) selon la revendication 7, **caractérisé en ce que** la tige d'extension d'instrument (3) est apte à être raccordée mécaniquement et/ou électriquement à la pièce à main (2) par l'intermédiaire du couplage (2a).

9. Instrument médical (1) selon l'une quelconque des revendications 7 à 8, **caractérisé en ce que** la pièce à main (2) présente une section d'extrémité distale au niveau de laquelle le couplage (2a) est disposé, dans lequel un logement d'outil (4) est disposé au niveau d'une section d'extrémité distale de la tige d'extension d'instrument (3).

10. Instrument médical (1) selon l'une quelconque des revendications 1 à 9, **caractérisé en ce que** la paire de paliers (6) forme une unité séparée destinée à être montée dans l'instrument médical (1) en incluant au moins la douille entretoise (6c) et de préférence le composant (11).

11. Instrument médical (1) selon l'une quelconque des revendications 1 à 9, **caractérisé en ce que** la douille entretoise (6c) est un élément fixe de l'instrument médical (1).

12. Instrument médical (1) selon l'une quelconque des revendications précédentes 1 à 11, **caractérisé en ce que** la bobine (13) est en forme de méandre ou de spirale ainsi que, vue dans la direction radiale de la douille entretoise (6c), en une ou plusieurs couches.

13. Instrument médical (1) selon l'une quelconque des revendications 1 à 12, **caractérisé en ce que** le composant (11), au niveau de la face d'enveloppe externe de celui-ci, présente un nombre d'évidements (11a) dans lesquels le nombre correspondant d'aimants permanents (12) est utilisé.

14. Instrument médical (1) selon l'une quelconque des revendications 1 à 13, **caractérisé en ce qu'**au moins la douille entretoise (6c) présente des lignes de signal radialement intérieures (14) qui sont reliées électriquement à la bobine (13) ainsi qu'à des lignes de signal radialement extérieures (14) qui sont couplées à des lignes de signal dans les paliers (6a, 6b) par l'intermédiaire de couplages électriques pour diriger ainsi des signaux électriques partant de la bobine (13) le long de l'instrument médical (1) vers le côté proximal.
